Europäisches Patentamt

European Patent Office

Office européen des brevets

(19)

(11) Veröffentlichungsnummer: **0 236 850**
**A2**

(12) # EUROPÄISCHE PATENTANMELDUNG

(21) Anmeldenummer: 87102666.2

(51) Int. Cl.⁴: **A61M 16/00 , F15B 1/00**

(22) Anmeldetag: **25.02.87**

(30) Priorität: **07.03.86 DE 3607488**

(43) Veröffentlichungstag der Anmeldung:
**16.09.87 Patentblatt 87/38**

(84) Benannte Vertragsstaaten:
**DE FR GB IT SE**

(71) Anmelder: **Drägerwerk Aktiengesellschaft**
**Moislinger Allee 53-55**
**D-2400 Lübeck 1(DE)**

(72) Erfinder: **Baumgarten, Siegfried**
**Huntenhorsten Weg 17d**
**D-2400 Lübeck(DE)**

(54) **Gasversorgungseinheit für pneumatisch betriebene Geräte.**

(57) Eine Gasversorgungseinheit für pneumatisch betriebene Geräte mit einer Gaszuführungsleitung und einer Reserveleitung, die bei einem Druckabfall in der Zuführungsleitung über ein ansteuerbares Schaltelement zuschaltbar ist, soll so verbessert werden, daß eine Umschaltung von Gaszuführungsleitungen auf Reserveleitungen und umgekehrt, oder eine wechselweise Umschaltung zwischen mehreren Gaszuführungsleitungen in eindeutige Schaltungszustände möglich ist. Dazu ist vorgesehen, daß die Zuführungsleitung (1) und die Reserveleitung (2) über ein gemeinsames Schaltelement (6) durch einen Aussetzregler (5) wechselweise an die Geräteleitung (3) anschaltbar sind.

EP 0 236 850 A2

## Gasversorgungseinheit für pneumatsich betriebene Geräte

Die Erfindung betrifft eine Gasversorgungseinheit für pneumatisch betriebene Geräte mit einer Gaszuführungsleitung und einer Reserveleitung, die bei einem Druckabfall in der Zuführungsleitung über ein ansteuerbares Schaltelement zuschaltbar ist.

Eine solche Gasversorgungseinheit ist bei einem Narkosebeatmungssystem in der DE-OS 29 45 485 beschrieben.

Bei der bekannten Gasversorgungseinheit wird ein atembares Frischgas über eine Anschlußleitung zu dem Beatmungsgerät geführt. Eine Druckleitung überwacht den Druck in der Frischgasleitung. Bei Druckabfall wird ein entsprechendes Ventil in einer Reserveleitung geöffnet, welche dadurch mit der Umgebungsluft in Verbindung gebracht wird. Statt des Frischgases mit unterschiedlicher Zusammensetzung wird in diesem Falle Umgebungsluft zu dem Beatmungsgerät ersatzweise geführt.

Derartige redundante Gaszuführungsleitungen sind bei allen solchen pneumatisch angetriebenen Geräten notwendig, welche lebenserhaltende Funktionen ausüben. Während des Betriebes muß dabei sichergestellt sein, daß bei Ausfall eines Antriebs- oder Versorgungsgases unmittelbar und fehlerfrei auf eine Ersatzgasversorgungsleitung umgeschaltet wird.

Eine derartige Umschaltung zeigt auch eine Gasmischvorrichtung für Narkosegeräte gemäß DE-OS 30 20 204. Dort ist eine Luftversorgungsleitung und eine Narkosegasversorgungsleitung jeweils über ein ansteuerbares Ventil absperrbar oder öffenbar. Bei Ausfall eines Gases wird das entsprechende Ventil geschlossen und das Ventil in der Reservegasleitung geöffnet.

Bei den bekannten Vorrichtungen ist es notwendig, überschneidungsfreie Ventile zu benutzen bzw. die einzelnen Ventile derart zu schalten, daß die erste Gaszuführungsleitung vollständig geschlossen ist, bevor die Reserveleitung geöffnet ist. Dadurch muß verhindert werden, daß eine Gasströmung während des Schaltvorganges aus der ersten Gaszuführungsleitung in die Reserveleitung und umgekehrt stattfindet. Bei der Verwendung von überschneidungsfreien Ventilen oder bei Durchführung der Wechselschaltung zwischen zwei oder mehreren Ventilen kann es im Fehlerfall zu Schaltstellungen kommen, bei welchen alle zuschaltbaren Leitungen im geschlossenen Zustand verharren. Dadurch würde das entsprechende pneumatisch betriebene Gerät ohne Funktion sein oder gänzlich ausfallen.

Die vorliegende Erfindung geht somit von der Aufgabenstellung aus, eine Gasversorgungseinheit für pneumatisch betriebene Geräte der genannten Art zu schaffen, die eine Umschaltung von Gaszuführungsleitungen auf Reserveleitungen und umgekehrt, oder eine wechselweise Umschaltung zwischen mehreren Gaszuführungsleitungen in eindeutige Schaltungszustände ermöglicht. Fehlerhafte Zwischenschaltstellungen sollen vermieden werden.

Die Lösung der Aufgabe erfolgt dadurch, daß die Zuführungsleitung und die Reserveleitung über ein gemeinsames Schaltelement durch einen Aussetzregler wechselweise an die Geräteleitung anschaltbar sind.

Die Vorteile der Erfindung liegen im wesentlichen darin, daß der Aussetzregler bei Erreichen einer vorgebbaren Druckschwelle das Schaltelement zwangsläufig in eine definierte Schaltstellung umschaltet, so daß eine eindeutige Zuordnung der verschiedenen Gasleitungen zueinander gewährleistet ist. Es können weiterhin überschneidungsfreie Ventile als Schaltelement benutzt werden, bei denen eine strömungslose Zwischenstellung vermieden ist. Eine Zusammenfassung mehrerer zu schaltender Gaszuführungsleitungen oder Reserveleitungen über ein gemeinsames Schaltelement verringert die Anzahl der notwendigen Baugruppen, ermöglicht einen einfachen Aufbau und erhöht die Funktionssicherheit des Schaltvorganges.

Ein Ausführungsbeispiel der Erfindung wird anhand der schematischen Zeichnung dargestellt und im folgenden näher erläutert.

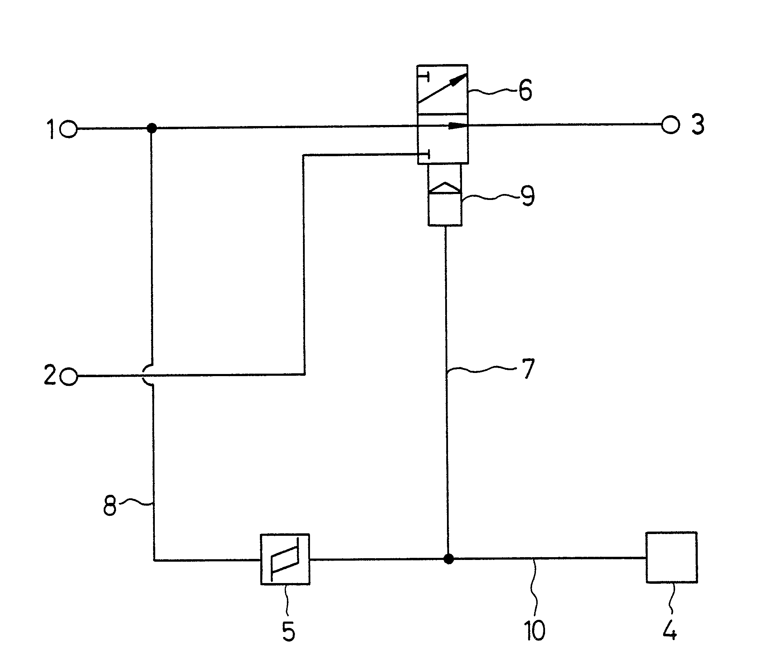

In der einzigen Figur ist eine Gasversorgungseinheit dargestellt, welche eine Druckgasleitung (1) als Gaszuführungsleitung und eine Druckgasleitung (2) als Reserveleitung besitzt. Beide Leitungen (1,2) können beispielsweise mit Sauerstoff betrieben sein. Ein Schaltelement (6) verbindet die Leitungen (1,2) mit einer Geräteleitung (3), welche an ein nicht dargestelltes pneumatisch betriebenes Gerät angeschlossen ist. Als Schaltelement (6) kann ein 3/2-Wegeventil eingesetzt werden, welches in der dargestellten Form eine Schaltstellung einnimmt, in der die Gaszuführungsleitung (1) mit der Geräteleitung (3) verbunden ist. Ein Aussetzregler (5) ist über eine Steuerleitung (7) an dem Steuerteil (9) des 3/2-Wegeventils (6), und über eine Druckleitung (8) an die Gaszuführungsleitung - (1) angeschlossen. Eine Anzeigeeinheit (4) ist über die Anzeigeleitung (10) mit der Steuerleitung (7) verbunden.

Im normalen Betriebsfall herrscht in der Zuführungsleitung (1) ein bestimmter Betriebsdruck, durch welchen der Aussetzregler über die Leitungen (7,8) das pneumatisch betätigte 3/2-Wegeventil (6) in der dargestellten Schaltstellung hält. Das Gas aus der Gaszuführungsleitung (1) kann unmittelbar in die Geräteleitung (3) strömen. Bei Druckabfall in der Gaszuführungsleitung (1) unter einem vorgebbaren Grenzwert, der durch den Aussetzregler (5) festgelegt ist, wird die Steuerleitung (7) entlüftet und damit das 3/2-Wegeventil (6) geschaltet, so daß die Reserveleitung (2) mit der Geräteleitung (3) verbunden ist. Bei Wiederanstig des Druckes in der Zuführungsleitung (1) über einen weiteren, durch den Aussetzregler (5) vorgegebenen Schwellwert, wird die Steuerleitung (7) wiederum mit Druck belastet und das 3/2-Wegeventil (6) kehrt in den dargestellten Schaltzustand zurück. Der Schaltzustand des 3/2-Wegeventiles (6) kann durch ein geeignetes Anzeigeelement (4), beispielsweise ein pneumatisch betätigtes Schauzeichen, angezeigt und gegebenenfalls weiterverarbeitet werden.

## Ansprüche

Gasversorgungseinheit für pneumatisch betriebene Geräte mit einer Gaszuführungsleitung und einer Reserveleitung, die bei einem Druckabfall in der Zuführungsleitung über ein ansteuerbares Schaltelement zuschaltbar ist, dadurch gekennzeichnet, daß die Zuführungsleitung (1) und die Reserveleitung (2) über ein gemeinsames Schaltelement (6) durch einen Aussetzregler (5) wechselweise an die Geräteleitung (3) anschaltbar sind.